# EUROPÄISCHE PATENTANMELDUNG

(11) **EP 2 062 551 A2**
(43) Veröffentlichungstag der Anmeldung: **27.05.2009**
(21) Anmeldenummer: 09154434.6
(22) Anmeldetag: 27.02.2004
(51) Int. Cl.: A61F 2/16

(54) **Linsenaufnahme für eine Vorrichtung zum Einsetzen verformbarer Intraocularlinsen**

(62) Teilanmeldung aus: 04715327.5
(71) Anmelder: Advanced Vision Science, Inc., Goleta, CA 93117 (US)
(72) Erfinder: Die Erfindernennung liegt noch nicht vor
(74) Vertreter: Rehberg Hüppe + Partner

(57) **Zusammenfassung**

Die Erfindung betrifft eine Linsenaufnahme (1) für eine Vorrichtung zum Einsetzen verformbarer Intraocularlinsen. Mit der Linsenaufnahme wird eine Intraocularlinse aus einem entspannten Zustand in einen elastisch verformten Zustand gebracht, um mit Hilfe der Vorrichtung in ein Auge injiziert zu werden, wo die Intraocularlinse wieder ihren entspannten Zustand einnimmt. Die erfindungsgemäße Linsenaufnahme (1) enthält eine flexible Unterlage (8), die von einer offenen Lage, in der sie eine Intraocularlinse in deren entspanntem Zustand aufzunehmen bestimmt ist, in eine geschlossene Lage verformbar ist. In der geschlossenen Lage ist die Linsenaufnahme (1) dazu bestimmt, in die Vorrichtung eingesetzt zu werden. Die flexible Unterlage (8) weist zwei gegenüberliegende, verstärkte Randbereiche (9, 10) aufweist und ist zwischen der offenen Lage und der geschlossenen Lage elastisch verformbar. Die flexible Unterlage (8) ist in der offenen Lage entspannt. Für eine alternative Ausführungsform ist die flexible Unterlage (8) in der geschlossenen Lage entspannt.

## Beschreibung

Die Erfindung betrifft eine Linsenaufnahme für eine Vorrichtung zum Einsetzen verformbarer Intraocularlinsen, mit welcher eine Intraocularlinse aus einem entspannten Zustand in einen elastisch verformten Zustand gebracht wird, um mit Hilfe der Vorrichtung in ein Auge injiziert zu werden, wo sie wieder ihren entspannten Zustand einnimmt.

Vorrichtungen zum Einsetzen verformbarer Intraocularlinsen sind bekannt. Ihr Hauptzweck besteht generell darin, den zum Einsetzen einer Intraocularlinse notwendigen Schnitt im Auge möglichst klein halten zu können. Eine Schwierigkeit solcher Vorrichtungen besteht darin, die Intraocularlinse derart in einen elastisch verformten Zustand zu bringen, dass sie in diesem Zustand durch eine Kanüle in ein Auge injiziert werden kann. Das Patent US4681102 zeigt eine derartige Vorrichtung. Eine Linsenaufnahme weist dabei ein Scharnier auf, welches es erlaubt, die Linsenaufnahme von einem offenen Zustand, in dem die Linse eingelegt wird, in einen geschlossenen Zustand zu bringen, in welchem die Linse zusammengefaltet ist. Das sich in der Mitte der Linsenaufnahme befindende Scharnier behindert anfänglich das Verformen der Linse und es kann sogar vorkommen, dass die Linse zu Beginn des Verformungsvorgangs ausweicht und entgegengesetzt zur beabsichtigten Verformungsrichtung gebogen wird. Die Vorrichtung gemäss US5947975 bringt diesbezüglich eine Verbesserung, indem die Linsenaufnahme zwei Scharniere aufweist. Beide der vorangehend erwähnten Vorrichtungen weisen den Nachteil auf, dass es umständlich ist, die Intraocularlinse in der Linsenaufnahme zu platzieren. Ein weiterer Nachteil dieser Vorrichtungen besteht darin, dass mit ihnen die Intraocularlinse praktisch um eine in Längsrichtung der Vorrichtung orientierte Linie gefaltet wird, was zu einer örtlichen Überbeanspruchung der Linse führen kann, wodurch diese unter Umständen dauernd verformt bleibt, insbesondere dann, wenn sie vor der Injektion zu lange in der Vorrichtung verweilte.

Ausgehend von diesem Stand der Technik liegen der Erfindung die Aufgaben zugrunde, eine Linsenaufnahme der eingangs genannten Art vorzuschlagen, bei welcher die Intraocularlinse einfach eingelegt werden kann und welche eine gleichmässige Verformung der Intraocularlinse ohne örtliche Überbeanspruchung ermöglicht.

Zur Lösung dieser Aufgaben enthält die Linsenaufnahme eine flexible Unterlage, die von einer offenen Lage, in der sie eine Intraocularlinse in deren entspanntem Zustand aufzunehmen bestimmt ist in eine geschlossene Lage verformbar ist, in der sie dazu bestimmt ist, in die Vorrichtung eingesetzt zu werden. Mit dem Begriff "flexible" Unterlage ist ein Bereich gemeint, der sich elastisch, teilplastisch oder plastisch verformen lässt, ohne dabei zu brechen. Durch diese Lösung ist die Intraocularlinse während des Vorgangs der Verformung gestützt, so dass sie nicht ausweichen oder sich verschieben kann. Zudem wird die Intraocularlinse über ihre gesamte Ausdehnung verformt und nicht wie beim Stand der Technik nur um eine oder zwei Biegeachsen. Dadurch wird eine örtliche Überbeanspruchung der Intraocularlinse zuverlässig vermieden.

Vorteilhaft ist die Linsenaufnahme so gestaltet, dass sie durch eine Biegung der flexiblen Unterlage von der offenen in die geschlossene Lage bringbar ist, wobei die flexible Unterlage und damit auch die mit ihr in Kontakt stehende Intraocularlinse einer zunehmenden Krümmung unterworfen wird. Dies erlaubt ein besonders schonendes Verformen der Intraocularlinse.

Nach einer Ausführungsart ist die flexible Unterlage zwischen der offenen Lage und der geschlossenen Lage elastisch verformbar, so dass sie jeweils wieder ihre Ausgangslage einnimmt, wenn sie von der Verformungskraft entlastet wird. Dabei kann die Linsenaufnahme entweder so gestaltet sein, dass sie in der offenen Lage entspannt ist oder dass sie in der geschlossenen Lage entspannt ist.

Nach einer Ausführungsart der Erfindung bildet die flexible Unterlage in der geschlossenen Lage einen Kanal zum Aufnehmen der verformten Intraocularlinse bildet. An diesen Kanal kann sich die genannte Kanüle anschliessen, in welche die verformte Intraocularlinse beispielsweise durch einen Stössel geschoben werden kann.

Nach einer weiteren Ausführungsart weist die flexible Unterlage zwei gegenüberliegende, verstärkte Randbereiche auf. Diese Randbereiche können mehrere Funktionen erfüllen, wie weiter unten noch klar werden wird. Beispielsweise kann beiderseits am Übergang von der flexiblen Unterlage zum jeweiligen Randbereich ein Hinterschnitt zum Halten und Führen der Ränder der Intraocularlinse vorhanden sein. Diese Randbereiche ermöglichen das genaue Positionieren der Intraocularlinse in der Linsenaufnahme und verhindern zudem das Verrutschen der Intraocularlinse während der Verformung. Vorteilhaft weist mindestens einer der hinterschnittenen Randbereiche eine Aussparung auf, damit beim Einlegen der Intraocularlinse deren Rand den Randbereich der Linsenaufnahme ungehindert passieren kann.

In der flexiblen Unterlage kann zudem eine Mulde zur Aufnahme des optischen Teils der Intraocularlinse angeordnet sein, wodurch das genaue Positionieren der Intraocularlinse in der Linsenaufnahme weiter erleichtert wird.

Nach einer weiteren Ausführungsart weist die flexible Unterlage an einem Ende zwischen den Randbereichen eine Verjüngung auf, um eine Führung für einen Stössel zum Transport der verformten Intraocularlinse zu bilden.

Gemäss einer anderen Ausführungsart hat die flexible Unterlage einen sich von der Mitte aus zu beiden Randbereichen hin stetig verändernden Querschnitt. Dadurch lässt sich eine vordefinierte Verformung der Linsenaufnahme erreichen, so dass die Biegelinie der Intraocularlinse optimiert werden kann.

Nach einer weiteren Ausführungsart sind in den Randbereichen der Linsenaufnahme Mittel zum gegenseitigen Verbinden der Randbereiche vorhanden. Diese Verbindungsmittel erlauben es, die Linsenaufnahme in ihrer geschlossenen Lage zu arretieren, was das Einsetzen der Linsenaufnahme mitsamt der darin aufgenommenen, verformten Intraocularlinse in eine entsprechende Injektionsvorrichtung erleichtert. Nach einer anderen Ausführungsart sind an den Randbereichen Greifmittel vorhanden, um das Verformen der Linsenaufnahme zu erleichtern.

Nach einer weiteren Ausführungsart wird der in der geschlossenen Lage gebildete Kanal zu einer Seite der Linsenaufnahme hin enger. Dies erlaubt es, die Linse durch ein Verschieben im Kanal weiter zu komprimieren, um sie schliesslich in die genannte Kanüle überzuführen, von der aus sie in ein Auge injiziert werden kann.

Gemäss einer weiteren Ausführungsart der Erfindung sind bei dem in der geschlossenen Lage gebildeten Kanal die Übergänge von der flexiblen Unterlage zu den Randbereichen so ausgebildet, dass der Kanal an einem seiner Enden einen schneckenartigen Querschnitt hat. Durch einen so geformten Kanal werden insbesondere grosse Intraocularlinsen während ihrer Verschiebung im Kanal so gerollt, dass sich ihre gegenüberliegenden Ränder überlappen.

Nach einer weitern Ausführungsart ist mindestens einer der Hinterschnitte zum einen Ende der Linsenaufnahme hin vergrössert, um einen Einlaufabschnitt für eine an der Intraocularlinse angeordnete Haptic zu bilden.

Noch eine Ausführungsart sieht vor, dass an der Linsenaufnahme Rastmittel vorhanden sind, um sie in einem Gehäuse der genannten Vorrichtung zu positionieren und zu halten.

Schliesslich besteht die Linsenaufnahme nach einer weiteren Ausführungsart aus Polypropylen und ist vorzugsweise im Spritzgiessverfahren einstückig hergestellt.

Ausführungsbeispiele der Erfindung werden nachstehend unter Bezugnahme auf die beiliegenden Zeichnungen beispielsweise beschrieben. Es zeigt:
- Figur 1: eine perspektivische Ansicht einer Vorrichtung zum Einsetzen ver- formbarer Intraocularlinsen mit einem Ausführungsbeispiel einer er- findungsgemässen Linsenaufnahme,
- Figur 2: eine perspektivische Ansicht einer ersten Ausführungsart der Lin- senaufnahme in ihrer offenen Lage,
- Figur 3: eine perspektivische Ansicht der Linsenaufnahme nach Figur 2 in ihrer geschlossenen Lage,
- Figur 4: eine perspektivische Ansicht einer zweiten Ausführungsart der Lin- senaufnahme in ihrer offenen Lage,
- Figur 5: eine perspektivische Ansicht der Linsenaufnahme nach Figur 4 in ihrer geschlossenen Lage und
- Figur 6: eine perspektivische Ansicht einer dritten Ausführungsart der Lin- senaufnahme in ihrer geschlossenen Lage, eingebaut in einer Vor- richtung.

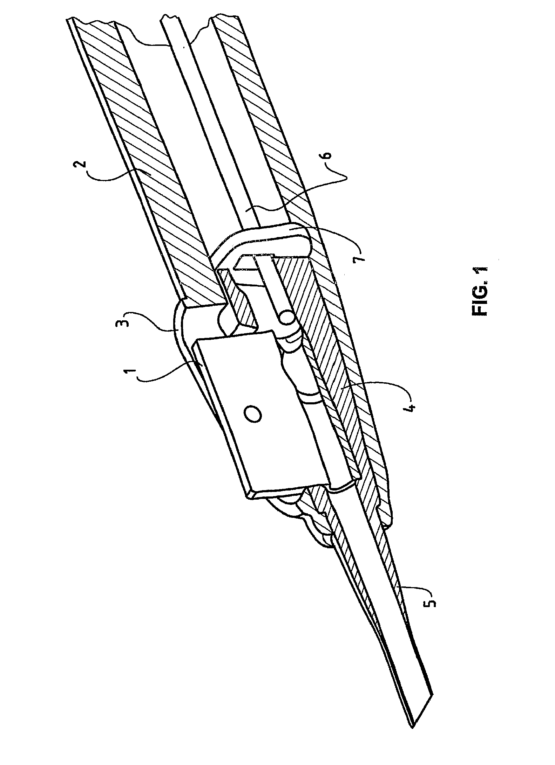

Figur 1 zeigt einen Ausschnitt aus einer Vorrichtung zum Einsetzen verformbarer Intraocularlinsen mit einer in der Vorrichtung eingesetzten Linsenaufnahme 1. Die Vorrichtung weist ein längliches Gehäuse 2 auf, in dessen Seitenwand eine längliche Öffnung 3 vorgesehen ist, die zum Einsetzen der Linsenaufnahme 1 dient. Im vorliegenden Beispiel ist die Linsenaufnahme 1 in einem Lagerteil 4 gehalten, wobei dieser Lagerteil 4 mit einer Kanüle 5 einstückig verbunden ist. Beim Gebrauch der Vorrichtung wird die Linsenaufnahme 1 mit einer darin im verformten Zustand aufgenommenen Intraocularlinse durch die Öffnung 3 in die Vorrichtung eingesetzt. Dann wird die Intraocularlinse durch einen Stössel 6 aus der Linsenaufnahme 1 in die Kanüle 5 vorgeschoben. Anschliessend wird die Kanüle 5 durch einen kleinen Einschnitt in das Auge eines Patienten eingesetzt und die Intraocularlinse wird durch den Stössel 6 aus der Kanüle 5 in das Auge geschoben. Ein im Gehäuse 2 angeordneter Führungsteil 7 sorgt dafür, dass der Stössel 6 die Intraocularlinse präzise erfasst.

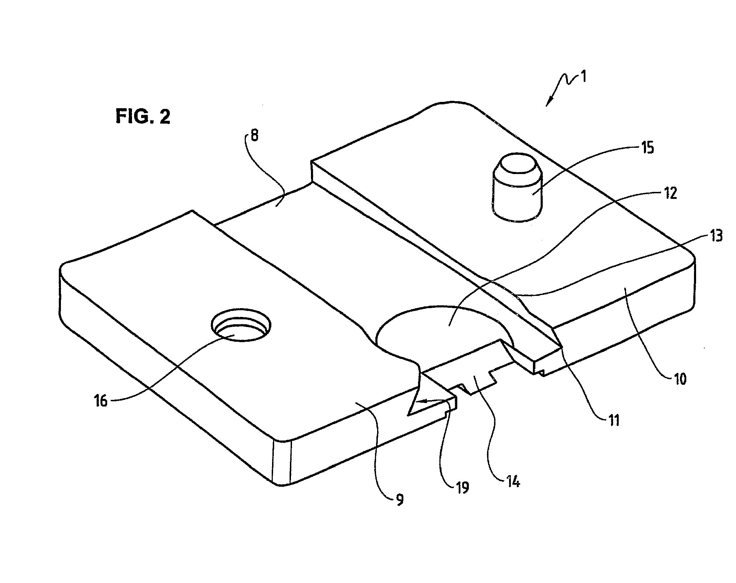

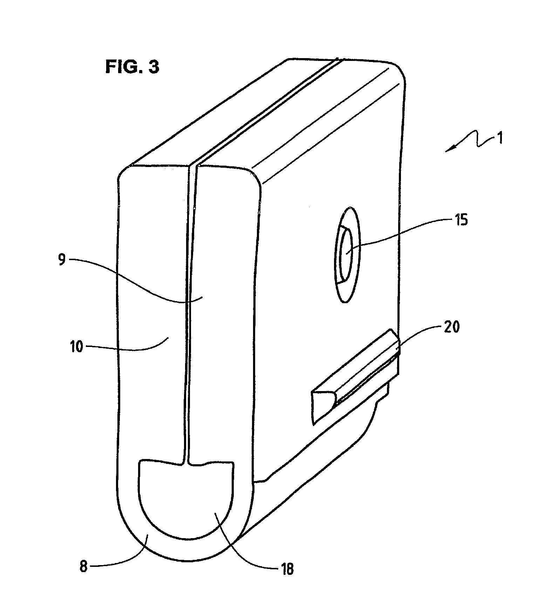

Figur 2 zeigt eine perspektivische Ansicht einer ersten Ausführungsart der Linsenaufnahme 1 in ihrer zur Aufnahme einer Intraocularlinse (nicht dargestellt) bereiten, offenen Lage. Die Linsenaufnahme 1 weist eine flexible Unterlage 8 auf, welche in dieser Lage im Wesentlichen eben ist. Beiderseits der flexiblen Unterlage 8 weist die Linsenaufnahme 1 Randbereiche 9 und 10 auf, die dicker und dadurch steifer sind als die flexible Unterlage 8. Am Übergang zwischen der flexiblen Unterlage zum Randbereich ist jeweils ein Hinterschnitt 11 vorhanden, der es erlaubt, die Ränder der einzulegenden Intraocularlinse während des Verformungsvorgangs zu halten und bei der Verschiebung in die Kanüle 5 zu führen. Um das Einlegen der Intraocularlinse zu erleichtern, ist in beiden Randbereichen jeweils eine Aussparung 13 angeordnet, die jedoch nicht bis zum Grund des Hinterschnitts 11 reicht. Eine in der flexiblen Unterlage 8 angeordnete Mulde 12 kann das Platzieren der Intraocularlinse weiter erleichtern. Bekannte Intraocularlinsen weisen zwei so genannte Haptics auf. Dies sind kleine Bügel, welche die Linse in der Linsentasche im Auge zentrieren. Um solche Intraocularlinsen mit der erfindungsgemässen Linsenaufnahme einzusetzen, weist die Linsenaufnahme 1 mit Vorteil im Bereich eines der Hinterschnitte 11 eine Erweiterung 19 auf, welche eine Haptic aufnimmt und verhindert, dass dieser später beim Verschieben der Intraocularlinse durch den Stössei 6 an der Linsenaufnahme 1 hängen bleibt. Wenn eine Intraocularlinse in der soeben beschriebenen Art in der Linsenaufnahme 1 platziert wurde, wird diese an ihren beiden Randbereichen 9 und 10 ergriffen und mitsamt der auf der flexiblen Unterlage 8 aufliegenden Intraocularlinse verformt, bis sie die in Figur 3 dargestellte Lage einnimmt. Eine Verjüngung 14 ist an einer Seite der flexiblen Unterlage 8 angeordnet und hat den Zweck, nach dem Verformen und dem Einsetzen in die Vorrichtung den Stössel 6 derart zu führen, dass er genau den Rand der verformten Intraocularlinse trifft, wenn diese aus der Linsenaufnahme 1 in die Kanüle 5 geschoben werden soll.

Figur 3 zeigt eine perspektivische Ansicht der Linsenaufnahme 1 gemäss Figur 2 in ihrer geschlossenen Lage. Deutlich ist ein Kanal 18 zu sehen, der durch die gebogene flexible Unterlage 8 und die Randbereiche 9 und 10 begrenzt ist. Damit die Linsenaufnahme 1 bis zum Einsetzen in die Vorrichtung in ihrer in Figur 3 dargestellten geschlossenen Lage verbleibt, sind Verbindungsmittel vorgesehen, die im vorliegenden Beispiel aus Zapfen 15 bestehen, die am Randbereich 10 vorhanden sind und in Öffnungen 16 eingreifen, die im Randbereich 9 angeordnet sind. An mindestens einer Seite der geschlossenen Linsenaufnahme sind Rastmittel, beispielsweise in der Form einer Rippe 20 angeordnet, welche dazu bestimmt sind, die Linsenaufnahme 1 im Gehäuse 2 der Vorrichtung genau zu positionieren und zu arretieren.

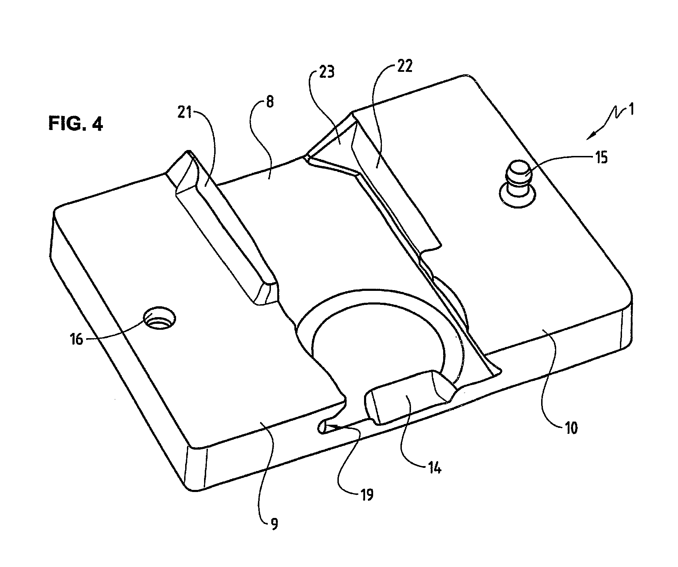

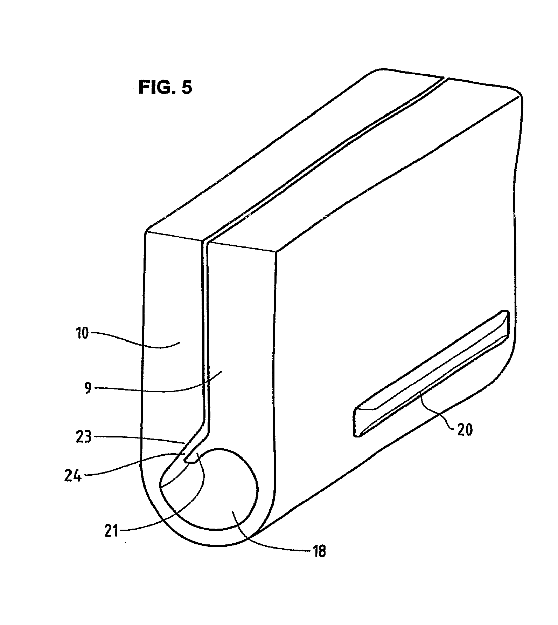

Die Figuren 4 und 5 zeigen eine andere Ausführungsart der Linsenaufnahme 1, welche speziell zum Injizieren von relativ grossen Intraocularlinsen vorgesehen ist. Figur 4 zeigt dabei die geöffnete Lage der Linsenaufnahme 1 und Figur 5 die geschlossene Lage. Für gleiche Teile wie in den Figuren 1 bis 3 sind gleiche Bezugszeichen verwendet. Die Linsenaufnahme 1 nach den Figuren 4 und 5 unterscheidet sich von jener nach den Figuren 2 und 3 insbesondere durch eine Erhebung 21, die im Randbereich 9 angrenzend an die flexible Unterlage 8 angeordnet ist. Im gegenüberliegenden Randbereich 10 ist eine Ausnehmung 22 vorgesehen, in welcher die Erhebung 21 in der geschlossenen Lage der Linsenaufnahme 1 Platz findet. In Figur 5 ist deutlich zu sehen, dass der Kanal 18 an der Austrittsseite durch die Erhebung 21 und die Ausnehmung 22 einen schneckenartigen Querschnitt erhält. Zwischen der Erhebung 21 und der Ausnehmung 22 ist dabei ein Spalt 24 vorhanden, der es erlaubt, eine grosse Intraocularlinse derart zu verformen, dass sich deren gegenüberliegende Ränder überschneiden, wodurch die Intraocularlinse die Linsenaufnahme 1 in einem aufgerollten Zustand verlässt. Um die derart aufgerollte Intraocularlinse in die Kanüle 5 überzuführen, ist im Randbereich 10 ein Leitteil 23 angeordnet. Bei dieser Ausführungsart sind die Zapfen 15 hinterschnitten, um in den Öffnungen 16 eine Rastwirkung zu erzielen.

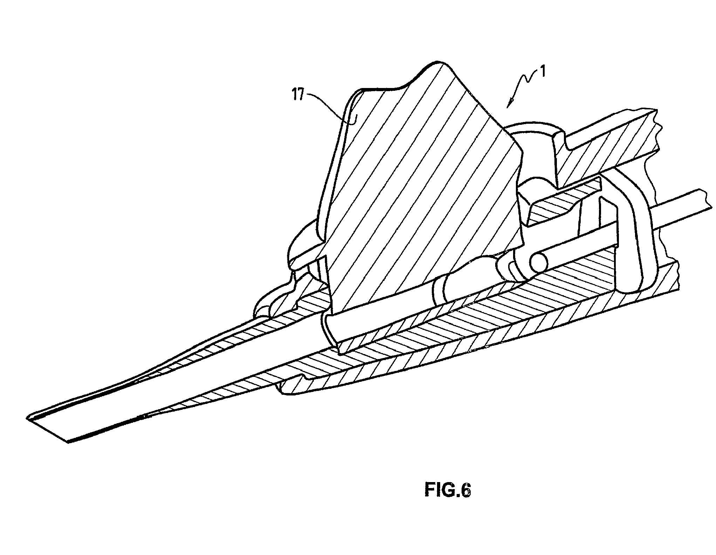

Bei der Ausführungsart nach Figur 6 sind an den Randbereichen 9 und 10 flügelartige Griffe 17 angeformt, damit sich die betreffende Linsenaufnahme besser ergreifen, verformen und halten lässt.

Gemäss einer weiteren, nicht zeichnerisch dargestellten Ausführungsart der Erfindung kann die flexible Unterlage eine Schlaufe bilden, welche eine Intraocularlinse in ihrem entspannten Zustand aufnimmt und dann zusammengezogen wird, beispielsweise etwa so, wie dies bei Kabelbindern der Fall ist.

## Patentansprüche

1. Linsenaufnahme (1) für eine Vorrichtung zum Einsetzen verformbarer Intraocularlinsen, mit welcher eine Intraocularlinse aus einem entspannten Zustand in einen elastisch verformten Zustand gebracht wird, um mit Hilfe der Vorrichtung in ein Auge injiziert zu werden, wo die Intraocularlinse wieder ihren entspannten Zustand einnimmt, wobei
a) die Linsenaufnahme (1) eine flexible Unterlage (8) enthält, die von einer offenen Lage, in der sie eine Intraocularlinse in deren entspanntem Zustand aufzunehmen bestimmt ist, in eine geschlossene Lage verformbar ist, in der sie dazu bestimmt ist, in die Vorrichtung eingesetzt zu werden,
b) die flexible Unterlage (8) zwei gegenüberliegende, verstärkte Randbereiche (9, 10) aufweist und
c) die flexible Unterlage (8) zwischen der offenen Lage und der geschlossenen Lage elastisch verformbar ist, wobei sie
ca) in der offenen Lage entspannt ist oder
cb) in der geschlossenen Lage entspannt ist.

2. Linsenaufnahme nach Anspruch 1, **dadurch gekennzeichnet, dass** sie derart ausgebildet ist, dass sie durch eine Biegung der flexiblen Unterlage (8) von der offenen in die geschlossene Lage bringbar ist, wobei die flexible Unterlage (8) und damit auch die mit ihr in Kontakt stehende Intraocularlinse einer zunehmenden Krümmung unterworfen wird.

3. Linsenaufnahme nach einem der vorangehenden Ansprüche, **dadurch gekennzeichnet, dass** die flexible Unterlage (8) in der geschlossenen Lage einen Kanal (18) zum Aufnehmen der verformten Intraocularlinse bildet.

4. Linsenaufnahme nach Anspruch 1, **dadurch gekennzeichnet, dass** beiderseits am Übergang von der flexiblen Unterlage zum jeweiligen Randbereich (9, 10) ein Hinterschnitt (11) zum Halten und Führen der Ränder der Intraocularlinse vorhanden ist.

5. Linsenaufnahme nach Anspruch 4, **dadurch gekennzeichnet, dass** mindestens einer der hinterschnittenen Randbereiche (9, 10) eine Aussparung (13) aufweist, damit beim Einlegen der Intraocularlinse deren Rand den Randbereich der Linsenaufnahme ungehindert passieren kann.

6. Linsenaufnahme nach einem der vorangehenden Ansprüche, **dadurch gekennzeichnet, dass** in der flexiblen Unterlage eine Mulde (12) zur Aufnahme des optischen Teils der Intraocularlinse angeordnet ist.

7. Linsenaufnahme nach einem der vorhergehenden Ansprüche, **dadurch gekennzeichnet, dass** die flexible Unterlage (8) an einem Ende zwischen den Randbereichen (9, 10) eine Verjüngung (14) aufweist, um eine Führung für einen Stößel (6) zum Transport der verformten Intraocularlinse zu bilden.

8. Linsenaufnahme nach einem der vorhergehenden Ansprüche, **dadurch gekennzeichnet, dass** die flexible Unterlage (8) einen sich von der Mitte aus zu beiden Randbereichen (9, 10) hin stetig verändernden Querschnitt hat.

9. Linsenaufnahme nach einem der vorhergehenden Ansprüche, **dadurch gekennzeichnet, dass** in den Randbereichen (9, 10) Mittel (15, 16) zum gegenseitigen Verbinden der Randbereiche vorhanden sind.

10. Linsenaufnahme nach einem der vorhergehenden Ansprüche, **dadurch gekennzeichnet, dass** an den Randbereichen Greifmittel (17) vorhanden sind, um das Verformen der flexiblen Unterlage (8) zu erleichtern.

11. Linsenaufnahme nach Anspruch 3, **dadurch gekennzeichnet, dass** der in der geschlossenen Lage gebildete Kanal (18) zu einer Seite der Linsenaufnahme hin enger wird.

12. Linsenaufnahme nach Anspruch 3 oder 11, **dadurch gekennzeichnet, dass** bei dem in der geschlossenen Lage gebildeten Kanal (18) die Übergänge von der flexiblen Unterlage zu den Randbereichen (9, 10) so ausgebildet sind, dass der Kanal an einem seiner Enden einen schneckenartigen Querschnitt hat.

13. Linsenaufnahme nach einem der vorhergehenden Ansprüche, **dadurch gekennzeichnet, dass** mindestens einer der Hinterschnitte (11) zum einen Ende der Linsenaufnahme hin vergrößert ist, um einen Einlaufabschnitt (19) für eine an der Intraocularlinse angeordnete Haptic zu bilden.

14. Linsenaufnahme nach einem der vorangehenden Ansprüche, **dadurch gekennzeichnet, dass** an ihr Rastmittel (20) vorhanden sind, um die Linsenaufnahme in einem Gehäuse (2) der genannten Vorrichtung zu positionieren und zu halten.

15. Linsenaufnahme nach einem der vorangehenden Ansprüche, **dadurch gekennzeichnet, dass** sie aus Polypropylen besteht und vorzugsweise im Spritzgießverfahren einstückig hergestellt ist.

16. Linsenaufnahme nach einem der vorhergehenden Ansprüche, **dadurch gekennzeichnet, dass** an den Randbereichen (9, 10) flügelartige Griffe (17) angeformt sind, über die sich die Linsenaufnahme besser ergreifen, verformen und halten lässt.
